# EUROPEAN PATENT APPLICATION

(11) **EP 4 588 498 A1**
(43) Date of publication of application: **23.07.2025**
(21) Application number: 23864661.6
(22) Date of filing: 11.09.2023
(51) Int. Cl.: A61M 25/00, A61B 5/00, A61B 5/02, A61B 18/12, A61B 18/14, A61M 31/00, A61N 5/067, A61N 7/00

(54) **MEDICAL INTERVENTIONAL CATHETER**

(30) Priority: 15.09.2022 CN 202211124333
(71) Applicant: Shanghai MicroPort Medical (Group) Co., Ltd., Shanghai 201203 (CN)
(72) Inventor: ZHOU, Qi, Shanghai 201203 (CN); ZHANG, Zhenghai, Shanghai 201203 (CN); YUE, Bin, Shanghai 201203 (CN)
(74) Representative: Lavoix
(86) International application number: PCT/CN2023/118008
(87) International publication number: WO 2024/055929

(57) **Abstract**

The present invention relates to a medical interventional catheter, which includes a catheter body with both diagnostic and therapeutic capabilities. The catheter body includes a distal functional member configured for image-based monitoring of a tubular lumen of interest and for release of a therapeutic source to a target location in the tubular lumen of interest. The therapeutic source includes therapeutic energy and/or a therapeutic substance. This interventional catheter with both diagnostic and therapeutic capabilities provides real-time image-based monitoring in a therapeutic procedure, making the procedure more accurate and effective.

## Description

### TECHNICAL FIELD

The present invention relates to the field of medical devices and, in particular, to a medical interventional catheter with both diagnostic and therapeutic capabilities.

### BACKGROUND

Atherosclerosis, a syndrome that affects arterial blood vessels, develops as a chronic inflammatory response in arterial walls, in large part due to plaques in arterial walls resulting from deposits of lipids, macrophages and foam cells. Atherosclerosis is commonly referred to arteriosclerosis, and its pathophysiology manifests as several types of lesions, ranging from fibrous, to lipidic, to calcified. In the current clinical practice, established therapeutic options for atherosclerosis mainly include medication, intervention and bypass surgery. However, all these approaches are associated with a number of problems including restenosis and thrombosis over time.

Thermo-physiotherapy has been widely used in clinical practice thanks to relatively low cost, few side effects and short treatment time. Thermo-physiotherapy is accomplished by thermal ablation, and available energy source options for thermal ablation primarily include cryoballoons, focused ultrasound waves, laser light and radio frequency (RF) radiation. Clinical studies have shown that RF ablation can provide a range of advantages, including determined safe frequencies, controllable thermal energy output and ease of functional integration. In order to ensure good thermo-physiotherapeutic performance, ablation-induced tissue damage or destruction must be limited in a determined range. However, at present, techniques remain absent for accurate ablation range monitoring and control for fibrous plaques.

Intravascular imaging is a mainstream monitoring technique, which majorly involves optical coherence tomography (OCT), intravascular ultrasound (IVUS), angioscopy, intravascular magnetic resonance imaging (MRI), etc. Compared with other imaging techniques, OCT is profoundly superior in resolution (higher than 10 µm) and can be used to capture high-definition images of biological tissues, which are very helpful in accurate imaging and identification of plaques within blood vessels. However, in therapeutic procedures, image-based diagnosis is typically separate from therapeutic treatment. This makes the procedures cumbersome, incapable of allowing real-time monitoring and not immediately available. In some circumstances, a high recurrence rate, as well as high overall therapeutic cost, would be expected.

### SUMMARY

It is an objective of the present invention to provide a medical interventional catheter with both diagnostic and therapeutic capabilities, which provides real-time image-based monitoring in a therapeutic procedure, making the procedure more accurate and effective.

To this end, the present invention provides a medical interventional catheter, which includes a catheter body with both diagnostic and therapeutic capabilities. The catheter body includes a functional member at a distal end thereof, the functional member configured for image-based monitoring of a tubular lumen of interest and for release of a therapeutic source to a target location in the tubular lumen of interest. The therapeutic source includes therapeutic energy and/or a therapeutic substance.

In one embodiment, the interventional catheter may further include a tip, wherein a distal end of the functional member is connected to a proximal end of the tip, and sealed, by an elastic coupling portion, and the tip defines a guidewire lumen.

In one embodiment, the functional member may accomplish the image-based monitoring of the tubular lumen of interest using one or more imaging approaches.

In one embodiment, the functional member may accomplish the image-based monitoring of the tubular lumen of interest using optical coherence tomography (OCT) imaging.

In one embodiment, the functional member may include an imaging probe and a transparent imaging window, the imaging probe disposed at the transparent imaging window.

In one embodiment, the functional member may be able to release one or more types of therapeutic energy.

In one embodiment, the functional member may be able to release at least one of radio frequency (RF) radiation, ultrasound waves, laser light and a cryofluid as the therapeutic energy.

In one embodiment, the functional member may accomplish the release of the therapeutic substance to the target location in the tubular lumen of interest using one or more approaches.

In one embodiment, the therapeutic substance may be a drug, wherein the functional member accomplishes the release of the therapeutic substance to the target location in the tubular lumen of interest using at least one of a drug-containing coating, a drug-delivery reservoir and drug-delivery microneedles.

In one embodiment, the functional member may employ a drug-delivery reservoir and/or drug-delivery microneedles, wherein the catheter body further includes a tubular body having an outer surface, on which the drug-delivery reservoir and/or the drug-delivery microneedles is/are provided, and defining therein a drug-delivery channel extending along an axis of the tubular body of the functional member to a proximal end of the tubular body and distally connected to the drug-delivery reservoir and/or the drug-delivery microneedles.

In one embodiment, the functional member may include an imaging probe, an electrode and a temperature-measuring element, the imaging probe configured to accomplish the image-based monitoring, the electrode configured to release RF radiation, the temperature-measuring element disposed on the electrode and configured to monitor a surface temperature of the electrode.

In one embodiment, the catheter body may further include a tubular body, wherein the imaging probe is provided in the tubular body, and the electrode is disposed on an outer surface of the tubular body,
the tubular body defines an imaging channel, an electrode lead channel and a temperature-control lead channel therein, which are independent of one another and each extend along an axis of the tubular body of the functional member to a proximal end of the tubular body, the imaging channel arranged at a center of the tubular body, the electrode lead channel and the temperature-control lead channel both arranged around the imaging channel,
the imaging channel provided therein with an imaging transmission structure connected to the imaging probe, the electrode lead channel provided therein with an electrode lead connected to the electrode, the temperature-control lead channel provided therein with a temperature-control lead connected to the temperature-measuring element.

In one embodiment, the tubular body may further define a temperature-control fluid channel therein, which is independent of the imaging channel, the electrode lead channel and the temperature-control lead channel and arranged beside the imaging channel, wherein the electrode defines temperature-control fluid output holes, and the temperature-control fluid channel extends from the proximal end of the tubular body along the axis thereof and is connected to the temperature-control fluid output holes and configured for transfer of a temperature-control fluid and for release of the temperature-control fluid to the target location through the temperature-control fluid output holes.

In one embodiment, the tubular body may be a braided single-lumen tube housing therein a temperature-control fluid tube, an imaging tube, an electrode lead tube and a temperature-control lead tube, the temperature-control fluid tube defining a lumen providing the temperature-control fluid channel, the imaging tube defining a lumen providing the imaging channel, the electrode lead tube defining a lumen providing the electrode lead channel, the temperature-control lead tube defining a lumen providing the temperature-control lead channel, each of the temperature-control fluid tube, the imaging tube, the electrode lead tube and the temperature-control lead tube having a wall thickness less than 0.2 mm.

In one embodiment, the temperature-control fluid tube, the imaging tube, the electrode lead tube and the temperature-control lead tube may be securely bonded together.

In one embodiment, the tubular body may have an outer diameter of 1.0 mm to 3.0 mm, the imaging channel may have a diameter not exceeding 1.0 mm, the temperature-control fluid channel may have a diameter not exceeding 0.5 mm, the electrode lead channel may have a diameter of 0.1 mm to 0.5 mm, and the temperature-control lead channel may have a diameter of 0.1 mm to 0.5 mm.

In one embodiment, the temperature-control fluid output holes may be micro-holes with a diameter of 50 µm to 200 µm.

In one embodiment, a plurality of electrodes may be spaced axially and/or circumferentially with respect to the catheter body, at least two of which are annular and spaced axially with respect to the catheter body, wherein the imaging probe is provided between adjacent two of the annular electrodes.

In one embodiment, the interventional catheter may further include an interface portion, to which a proximal end of the catheter body is connected, and which includes:
an imaging interface connected to a proximal end of the imaging transmission structure;
a fluidic interface connected to a proximal end of the temperature-control fluid channel; and
an electrical interface connected to proximal ends of the electrode lead and the temperature-control lead.

In one embodiment, the functional member may include an imaging probe configured to accomplish the image-based monitoring, wherein the catheter body further includes a tubular body, in which the imaging probe is disposed, the tubular body defining therein an imaging channel extending along an axis of the tubular body of the functional member to a proximal end of the tubular body, the imaging channel provided therein with an imaging transmission structure connected to the imaging probe, the imaging transmission structure configured to be driven by a driving member to rotate the imaging probe circumferentially and/or translate it axially with respect to the catheter body.

In one embodiment, the imaging transmission structure may include an imaging optical fiber, a protective tube and a torsion spring, the protective tube disposed over the imaging optical fiber, the torsion spring disposed between the protective tube and the imaging optical fiber, the imaging optical fiber coupled to the imaging probe at one end and to the driving member at the other end.

In the proposed medical interventional catheter, the distal functional member in the catheter body is capable of image-based monitoring of a tubular lumen of interest (e.g., a blood vessel), and of releasing a therapeutic source to a target location in the tubular lumen of interest. The therapeutic source may include therapeutic energy and/or a therapeutic substance. With this arrangement, the interventional catheter can function as both an imaging catheter and a therapeutic catheter. Through combining diagnostic and therapeutic capabilities in a single catheter, interventional treatment of a lesion can be carried out without exchange between an imaging catheter and a therapeutic catheter, which may require relocating the site in need of therapeutic treatment, thereby simplifying the surgical procedure and making it easier to carry on. Moreover, a therapeutic effect of the procedure can be monitored in real time based on captured images, making it more accurate and effective, increasing its success rate and reducing the time that it requires.

### BRIEF DESCRIPTION OF THE DRAWINGS

Those of ordinary skill in the art will understand that the following drawings are presented to enable a better understanding of the present invention and not intended to limit the scope thereof in any sense, in which:
Fig. 1 is a schematic diagram showing the overall structure of a medical interventional catheter according to an embodiment of the present invention;
Fig. 2 is a schematic diagram showing the structure of a medical interventional catheter according to an embodiment of the present invention in detail;
Fig. 3 is a schematic diagram showing the structure of a medical interventional catheter according to a first embodiment of the present invention in detail, which is being used as an ablation catheter;
Fig. 4 is an enlarged view of portion a of the interventional catheter of Fig. 3;
Fig. 5a is a cross-sectional view of the structure of Fig. 4 taken along line A-A;
Fig. 5b is a cross-sectional view of the structure of Fig. 4 taken along line B-B; and
Fig. 5c is a cross-sectional view of the structure of Fig. 4 taken along line C-C.

### DETAILED DESCRIPTION

Objectives, advantages and features of the present invention will become more apparent upon reading the following description with reference to the accompanying drawings. Note that the figures are provided in a very simplified form not necessarily drawn to exact scale for the only purpose of helping to explain the disclosed embodiments in a more convenient and clearer way.

As used herein, the singular forms "a", "an" and "the" include plural referents, unless the context clearly dictates otherwise. As used herein, the term "or" is generally employed in the sense of "and/or", unless the context clearly dictates otherwise. As used herein, the term "plurality" means "two or more", and the term "a number of" refers to a non-limited number, unless otherwise specified. The following description sets forth numerous specific details in order to provide a more thorough understanding of the present invention. However, it will be apparent to those skilled in the art that the present invention can be practiced without one or more of these specific details. In other instances, well-known technical features have not been described in order to avoid unnecessary obscuring of the invention.

In the following, for ease of illustration, the terms "distal", "proximal", "axial" and "circumferential" may be used. When used to describe a medical interventional catheter, "distal" refers to an end away from an operator who is operating the catheter, while "proximal" refers to an end closer to the operator. "Axial" refers to a direction defined along a central axis of the interventional catheter, and "circumferential" refers to a direction defined about the central axis. The central axis refers to a lengthwise direction of the interventional catheter.

The present invention will be further described below with reference to the accompanying drawings, which illustrate preferred embodiments thereof. If there is no conflict, the embodiments described hereunder and features thereof can complement or be combined with each other.

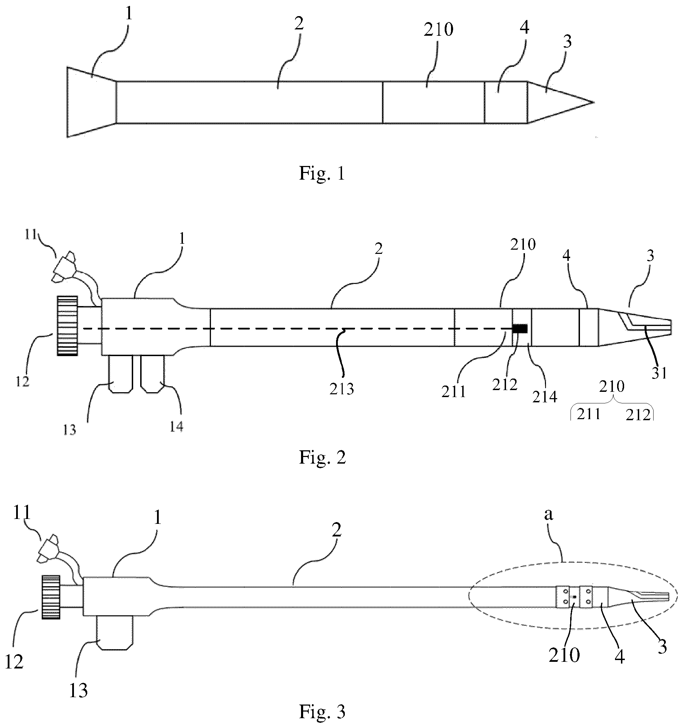

As shown in Figs. 1 and 2, in an embodiment of the present invention, there is provided a medical interventional catheter with both diagnostic and therapeutic capabilities. In principle, this interventional catheter is used for coronary intervention, such as interventional treatment of atherosclerosis. It can provide real-time image-based monitoring in a therapeutic procedure, making the procedure more accurate and effective.

According to embodiments disclosed herein, the interventional catheter includes a catheter body 2 capable of both diagnosis and therapeutic treatment. The catheter body 2 includes a distal functional member 210. The distal functional member 210 is used for image-based monitoring of a tubular lumen of interest and delivery of a therapeutic source to a target location in the tubular lumen of interest. The therapeutic source includes therapeutic energy and/or a therapeutic substance. Here, the "tubular lumen of interest" refers to a blood vessel, preferably a coronary artery.

The functional member 210 may employ one or more imaging techniques to provide image-based monitoring of the tubular lumen of interest, such as at least one of optical imaging and ultrasonic imaging, preferably optical coherence tomography (OCT), which provides the highest resolution among the currently available imaging techniques, helping in imaging and identification of an intravascular plaque.

The functional member 210 is able to deliver one or more types of therapeutic energy, the functional member 210 is able to deliver such as at least one of radio frequency (RF) radiation, ultrasound waves, laser light and a cryofluid.

The functional member 210 may employ one or more methods to deliver a therapeutic substance to the target location. Here, the therapeutic substance refers to a therapeutic agent, namely, a drug. The present invention is not limited to any particular type of drug, and any desired drug is possible, such as anti-proliferative, anti-hyperplastic, anti-restenotic, anti-inflammatory, antibacterial, anti-tumor, anti-mitotic, anti-metastatic, anti-thrombotic, anti-osteoporotic, anti-angiogenic, cytostatic or microtubule-inhibitory. The functional member 210 may deliver the drug to the target location from at least one of a drug-containing coating, a drug-delivery reservoir and drug-delivery microneedles.

Therefore, the interventional catheter proposed herein can function as both an imaging catheter and a therapeutic catheter. Through combining diagnostic and therapeutic capabilities in a single catheter, interventional treatment of a lesion can be carried out without exchange between an imaging catheter and a therapeutic catheter, which may require relocating the site in need of therapeutic treatment, thereby simplifying the surgical procedure and making it easier to carry on. Moreover, a therapeutic effect of the procedure can be monitored in real time based on captured images, making it more accurate and effective, increasing its success rate and reducing the time that it requires.

As shown in Figs. 1 and 2, in one embodiment, the functional member 210 includes a therapeutic component 211 and an imaging probe 212. The therapeutic component 211 is used to deliver a therapeutic source to the target location (including a lesion). The therapeutic component 211 may deliver at least one of RF radiation, ultrasound waves, laser light and a cryofluid as therapeutic energy. Additionally, or alternatively, the therapeutic component 211 may have one or more features capable of delivering a therapeutic substance to the target location.

In one embodiment, the therapeutic component 211 includes an energy output means for outputting therapeutic energy. The energy output means may be at least one of electrodes for outputting RF radiation, an ultrasonic transducer for producing ultrasound waves, a laser focusing lens for outputting laser light and a cryofluid passage sealed within the interventional catheter and configured to transmit energy necessary for cryoablation.

In one embodiment, the therapeutic component 211 includes a therapeutic agent output means for outputting a therapeutic agent to the target location. The therapeutic agent output means may be of any suitable structure. For example, it may include at least one of a drug-containing coating and a drug-releasing structure. The drug-releasing structure includes a drug-delivery reservoir and/or drug-delivery microneedles. A drug may be either pre-stored in, or delivered through, the drug-delivery microneedles.

In a specific example, the catheter body 2 further includes a tubular body, and the drug-containing coating, drug-delivery reservoir and/or drug-delivery microneedles is/are provided on an outer surface of the tubular body. That is, the functional member 210 itself includes the tubular body, and the drug-containing coating, drug-delivery reservoir and/or drug-delivery microneedles is/are provided on the outer surface of the tubular body. The therapeutic agent output means may employ either a single approach or a combination of two or more approaches to deliver the therapeutic agent. For example, both the drug-containing coating and the drug-releasing structure may be provided. It is to be noted that either or both the energy output means and the therapeutic agent output means may be provided. In addition, in case of the therapeutic agent output means being implemented as the drug-delivery reservoir and/or drug-delivery microneedles, the tubular body of the functional member 210 may define a drug-delivery channel extending along an axis of the tubular body to a proximal end thereof and distally connected to the drug-delivery reservoir and/or the drug-delivery microneedles.

The imaging probe 212 is configured for image-based intravascular monitoring, particularly of a diseased region, which facilitates pre-operative identification of locations and compositions of lesions, intra-operative monitoring of a therapeutic effect of the procedure or delivery of the therapeutic agent, and post-operative evaluation of therapeutic efficacy based on image scanning after the therapeutic treatment of the diseased region is completed. This application is not limited to any imaging method that the imaging probe 212 employs. The imaging probe 212 may adopt optical or ultrasonic imaging, preferably optical coherence tomography (OCT). The imaging probe 212 may be implemented as at least one of a microlens (focusing optic), an ultrasonic probe and an optical reflector. That is, the imaging probe 212 may employ a single imaging approach, or a combination of two or more imaging approaches.

As shown in Figs. 2 to 3, 4, 5a and 5b, the imaging probe 212 is provided in the tubular body, and the tubular body defines an internal imaging channel 21, the imaging channel 21 extends along the axis of the tubular body of the functional member 210 to the proximal end of the tubular body. An imaging transmission structure 213 is provided in the imaging channel 21 and coupled to the imaging probe 212. Preferably, the imaging transmission structure 213 is configured to be driven by a driving member to rotate the imaging probe 212 circumferentially and/or translate it axially with respect to the catheter body 2 to make adjustments in the position and orientation of the imaging probe 212. This enables more flexible and more convenient intravascular image-based monitoring.

Specifically, one end of the imaging transmission structure 213 is coupled to the imaging probe 212, and its other end is passed through the imaging channel 21 to the proximal end of the catheter body 2. Optionally, the other end of the imaging transmission structure 213 may be connected to an interface portion 1. The imaging transmission structure 213 is configured to transmit collected signals and energy necessary for imaging. In one embodiment, the imaging probe 212 employs optical imaging. In this case, the imaging transmission structure 213 includes an imaging optical fiber, and the imaging probe 212 is implemented as an optical probe. Preferably, the imaging transmission structure 213 further includes a protective tube and a torsion spring. The protective tube is disposed over the imaging optical fiber, the torsion spring is provided between the protective tube and the imaging optical fiber. One end of the imaging optical fiber is coupled to the imaging probe 212, and the other end thereof is configured to connect the driving member. The torsion spring is provided to facilitate transmission of torques for driving movement of the imaging optical fiber and the imaging probe 212.

In one embodiment, the imaging probe 212 employs an optical coherence tomography (OCT) technique, in which an imaging optical fiber with ultra-low propagation losses and an extremely small diameter (e.g., 200 µm) is used as a light guide medium, and a microlens is used as a focusing optic for collection and emission of optical signals. The focusing optic may be a spherical lens, or a gradient refractive index (GRIN) lens. Since the optical fiber is, per se, a fragile glass fiber, it is very easy to break if not protected during use. Accordingly, the optical fiber is entirely cladded with a protective tube to form an optical fiber cable. This can prevent mechanical damage to the optical fiber and the distal focusing optic during movement and impart to them increased resistance to tensile and flexing forces. The protective tube may be selected as a transparent tube.

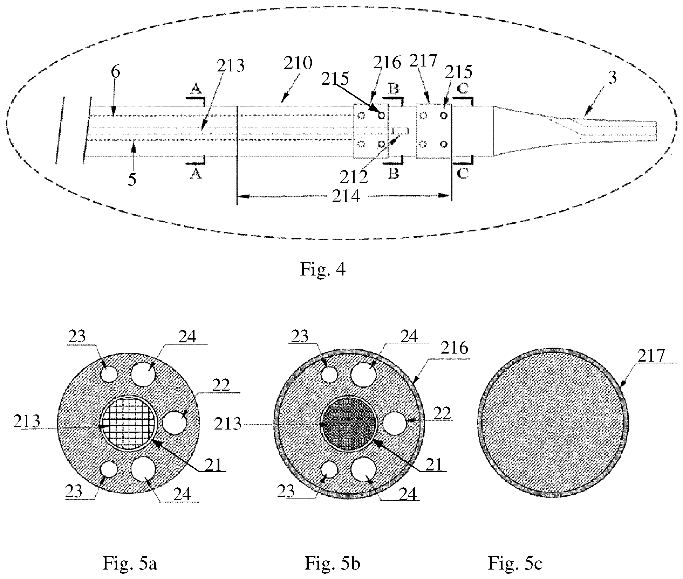

As shown in Figs. 2 and 4, the functional member 210 includes a transparent imaging window 214, and the imaging probe 212 is disposed in alignment with the imaging window 214. The imaging window 214 facilitates optical signal emission and reception of the imaging probe 212. The imaging window 214 may have an axial length of 2 mm to 100 mm, which is, for example, equal to an axial length of the tubular body measured from a proximal end of a coupling portion 4 toward a distal end thereof. Thus, it can be understood that the imaging window 214 is provided by a transparent portion of the tubular body of the functional member 210. The transparent portion may be made of nylon. The other portion of the tubular body than the imaging window 214 is essentially made of an opaque material, such as polyamide. Preferably, this portion is a braided tube.

As shown in Figs. 1 and 2, in one embodiment, the catheter body 2 is proximally coupled to the interface portion 1. The interface portion 1 is adapted for connection to an external device and for input and output of information. Information that can be input and output through the interface portion 1 includes at least energy and collected signals. The energy includes at least that necessary for image-based monitoring, and the collected signals include at least collected image signals. The interface portion 1 includes a number of interfaces. The number and types of interfaces may depend on the functionality of the functional member 210.

In one embodiment, the interface portion 1 includes a fluidic interface 11 for filling of a fluid, an imaging interface 12, an electrical interface 13 for transmission of electrical signals (e.g., electrical currents) and a mechanical interface 14 for transmission of mechanical power. The fluidic interface 11 is adapted for connection to a fluid filling device for filling a temperature-control fluid into the interventional catheter, which is typically normal solution. The imaging interface 12 is adapted for connection to an external imaging system capable of outputting energy necessary for imaging (e.g., laser radiation or electrical energy) to the interventional catheter and receiving collected signals from the interventional catheter. The electrical interface 13 is adapted for connection to an external energy output device for outputting electrical energy necessary for therapeutic treatment and temperature monitoring to the interventional catheter. The electrical interface 13 may also be connected to an external control device for receiving temperature signals from the interventional catheter. The mechanical interface 14 is adapted for connection to a driving member for driving the imaging probe 212 to rotate circumferentially and/or translate axially with respect to the catheter body 2. In one embodiment, the other end of the imaging transmission structure 213 is coupled to the mechanical interface 14. The mechanical interface 14 may be integrated with the imaging interface 12. Through the mechanical interface 14, the driving member can drive movement and rotation of the imaging transmission structure 213 and the imaging probe 212. The driving member may be selected as a motor.

As shown in Fig. 4, in conjunction with Figs. 5a and 5b, in a preferred embodiment, a central axis of the imaging channel 21 coincides with a central axis of the tubular body. That is, the imaging channel 21 is located at the center of the tubular body. The imaging channel 21 is the bulkiest component of the interventional catheter, and disposing it at the center of the catheter can enhance overall coaxiality of the catheter and facilitate the arrangement of its components. In particular, channels of other functions can be more easily arranged around the imaging channel 21, enabling the internal space of the tubular body to be efficiently utilized to ensure sufficient strength of the catheter body 2 without expanding an outer diameter of the interventional catheter. This facilitates access of the catheter to a narrow blood vessel in need of therapeutic intervention.

In addition to the imaging channel 21 for image-based monitoring, in some embodiments, the tubular body of the catheter body 2 may also house one or more channels for transmitting therapeutic energy and/or a therapeutic agent to the therapeutic component 211. This application is not limited to any particular number of channels within the tubular body, and any number of channels may be arranged therein if required.

The present application is also not limited to any particular form of the channels in the tubular body. For example, the tubular body may be itself a multi-lumen tube, and the channels may be provided by lumens of the multi-lumen tube. Alternatively, the tubular body may be a single-lumen tube, and the channels may be provided by individual tubes received in the lumen of the single-lumen tube. In each case, the tubular body should provide sufficiently strong support to the individual channels. Preferably, the single-lumen tube is a braided tube with sufficient strength and a relatively small wall thickness, which ensures that the lumen can provide a large enough accommodation space.

In a preferred embodiment, the functional member 210 includes the imaging probe 212, electrodes and temperature-measuring elements. In this case, the electrodes are included in the energy output means and configured to deliver RF radiation. The temperature-measuring elements are provided on the electrodes to monitor surface temperatures of the electrodes. In order to ensure overall compactness of the interventional catheter, it is necessary to appropriately arrange the imaging transmission structure 213 coupled to the imaging probe 212, temperature-control leads coupled to the temperature-measuring elements and electrode leads coupled to the electrodes.

In one embodiment, a plurality of electrodes are included, which are spaced axially and/or circumferentially with respect to the catheter body 2. The electrodes may be annular or not. In case of annular electrodes, the catheter is suitable for ablation of concentric, diffuse plaques. In case of non-annular electrodes, the catheter is suitable for ablation of eccentric plaques. Preferably, the proposed interventional catheter includes both annular and non-annular electrodes and is therefore suitable for geometrically-compatible therapeutic treatment of lesions, i.e., therapeutic treatment of lesions in various shapes. Thus, it can meet various clinical therapeutic needs.

The electrodes are attached to the outer surface of the tubular body of the functional member 210. The electrodes may be in the shape of rings or strips, and may be made as sheets or meshes. Without limitation, the electrodes may be radiopaque or not. The present application is not limited to any particular material, from which the electrodes are fabricated. For example, they may be RF electrodes made of a platinum-iridium alloy, platinum, copper, iron, stainless steel or the like. In addition, each electrode must be separated from any other adjacent electrode by a prescribed insulating distance, which should not be excessively small or excessively large. Pulsed electric fields are generated between positive and negative electrodes as electrode signals. If the inter-electrode insulating distance is too small, electric sparks and low-temperature plasmas will tend to occur. If the insulating distance is too large, the strength of the generated electric fields will be adversely affected. For these reasons, the inter-electrode insulating distance should not be arbitrary. Instead, it must ensure that the energy and strength of the generated electric fields will not cause ionization, thus guaranteeing the safety of energy delivered to a lesion. In one embodiment, the inter-electrode insulating distance is between 1 mm and 5 mm, which allows accurate ablation range control while preventing the occurrence of discharge due to an inadequate spacing between any two electrodes. The electrodes may have any required dimensions. In one embodiment, the electrodes may have a width of 2 mm to 10 mm measured along an axis of the interventional catheter and a thickness of 0.05 mm to 0.5 mm measured along a radial direction of the interventional catheter. It would be appreciated that the dimensions of the electrodes should depend on the size of a lesion in need of treatment. In general, larger electrodes mean a greater ablation range. Given the fact that most plaques have a size of around 1 mm, different therapeutic needs can be satisfied by configuring the axial width of the electrodes in the range of 2 mm to 10 mm.

The temperature-measuring elements are used to monitor a temperature at the target location, where therapeutic energy is delivered, to enable accurate strength control of the output therapeutic energy. The temperature-measuring elements are able to acquire more accurate temperature information of a lesion, which helps improve therapeutic efficacy. The temperature-measuring elements may be implemented as any suitable mechanisms, such as thermocouples, thermistors or lenses capable of collecting thermal signals. The temperature-measuring elements may be implemented as at least one of thermocouples, thermistors and lenses capable of collecting thermal signals. In one embodiment of the present application, the temperature-measuring elements are thermocouples, which directly monitor surface temperatures of the electrodes and determine the temperature of a lesion based on the surface temperatures of the electrodes.

Further, the electrodes are disposed on the outer surface of the tubular body. As shown in Figs. 5a and 5b, the tubular body defines the imaging channel 21, temperature-control lead channels 23 and electrode lead channels 24 are independent of one another. The tubular body defines the imaging channel 21, temperature-control lead channels 23 and electrode lead channels 24 each extend along the axis of the tubular body of the functional member 210 to the proximal end thereof. The imaging channel 21 is disposed at the center of the tubular body, and the electrode lead channels 24 and the temperature-control lead channels 23 are all arranged around the imaging channel 21. The electrode leads 6 coupled to the electrodes are received in the electrode lead channels 24, and the temperature-control leads 5 coupled to the temperature-measuring elements are received in the temperature-control lead channels 23. In an alternative embodiment, the temperature-control leads 5 and the electrode leads 6 may be disposed within a single lead channel. In this case, they may be provided with an insulating coating, which isolates them from one another to prevent crosstalk. The number of electrodes is equal to that of temperature-measuring elements. Each of the electrodes is coupled to a respective one of the electrode leads 6. One end of each electrode lead 6 is welded to an inner surface of the respective electrode, and the other end thereof is passed through a respective one of the electrode lead channels 24 and coupled to the electrical interface 13. The electrode leads 6 may be made of a material, as required. For example, the material of the electrode leads may be selected as one with a suitable resistance value per unit length. Each of the temperature-measuring elements is coupled to a respective one of the temperature-control leads 5. One end of each temperature-control lead 5 is coupled to the respective temperature-measuring element, and the other end thereof is passed through a respective one of the temperature-control lead channels 23 and coupled to the electrical interface 13. The electrode leads 6 and the temperature-control leads 5 may be coupled to the same electrical interface 13, or to different electrical interfaces 13. Preferably, the electrode leads 6 and the temperature-control leads 5 are sized on the order of several microns, which can contribute to easier control of the catheter's overall size.

In a modified embodiment, the tubular body further defines a temperature-control fluid channel 22 therein, the temperature-control fluid channel 22 is independent of the imaging channel 21, the temperature-control lead channels 23 and the electrode lead channels 24. That is, these channels are isolated from one another without mutual interference. The temperature-control fluid channel 22 is arranged beside the imaging channel 21, and the electrodes define temperature-control fluid output holes 215 therein. The temperature-control fluid channel 22 extends from the proximal end of the tubular body along the axis thereof and is connected to the temperature-control fluid output holes 215. The temperature-control fluid channel 22 is configured for transfer of the temperature-control fluid in such a manner that the fluid flows through the temperature-control fluid output holes 215 to the target location. Optionally, the proximal end of the temperature-control fluid channel 22 is connected to the interface portion 1, for example, to the fluidic interface 11. The temperature-control fluid output holes 215 are configured for release of the temperature-control fluid at a given temperature (e.g., a hot gas, or a cold saline solution) to the target location (e.g., a location in need of ablation), which can reduce damage to tissue due to excessive heat or cold. Thus, in an energy-based therapeutic procedure, the fluid can be delivered at a cold or hot temperature to the target location through the temperature-control fluid channel 22 to maintain temperatures of both the interventional catheter and a surface of tissue, with which the catheter is brought into contact, within the normal body temperature range, providing protection to unintended sites and increasing safety of the therapeutic procedure.

The temperature-control fluid output holes 215 are preferred to be micro-holes. An excessively large diameter of the micro-holes may affect the overall shape of the electrodes and hence the ablation efficacy. On the other hand, if the diameter of the micro-holes is too small, the fluid may be unable to flow out of the micro-holes depending on its viscosity. For these reasons, the diameter of the micro-holes is preferred to be 50 µm to 200 µm. The micro-holes are provided to reduce the influence of output of the temperature-control fluid on the electrodes. The temperature of the temperature-control fluid may be adjusted, as required, for example, within the range of 15-30 °C. The temperature of the temperature-control fluid and its output energy and power may be both adjustable to completely protect the endothelium from the ablation energy.

In one embodiment, the tubular body is a braided single-lumen tube, in which a temperature-control fluid tube, an imaging tube, electrode lead tubes and temperature-control lead tubes are arranged. A lumen of the temperature-control fluid tube provides the temperature-control fluid channel 22, and a lumen of the imaging tube provides the imaging channel 21. Lumens of the electrode lead tubes provide the electrode lead channels 24, and lumens of the temperature-control lead tubes provide the temperature-control lead channels 23. The temperature-control fluid tube, the imaging tube, the electrode lead tubes and the temperature-control lead tubes in the braided single-lumen tube may be spaced apart from one another. Alternatively, they may be brought into close contact, but not bonded to, one another. Still alternatively, they may be securely bonded, e.g., glued, to one another at their outer surfaces. The temperature-control fluid tube, the imaging tube, the electrode lead tubes and the temperature-control lead tubes may each have a wall thickness less than 0.2 mm, which helps reduce overall size of the interventional catheter and makes it easier to control.

In one specific application, the proposed interventional catheter is used to treat a coronary artery. In this case, its outer diameter is preferably 1.0 mm to 3.0 mm, more preferably 1.8 mm to 2.0 mm. If the outer diameter of the tubular body were larger than 3.0 mm, interventional catheter would be difficult to use for coronary intervention due to bulkiness. If the outer diameter of the tubular body were less than 1.0 mm, it would be difficult to integrate the aforementioned channels therein, and the manufacturing would encounter greater difficulties. The tubular body may have a wall thickness of 0.1 mm to 0.5 mm, while ensures both sufficient overall strength and good compliance of the interventional catheter. Further, the imaging channel 21 has a diameter not exceeding 1.0 mm, and the temperature-control fluid channel 22 has a diameter not exceeding 0.5 mm. Each electrode lead channel 24 has a diameter of 0.1 mm to 0.5 mm, and each temperature-control lead channel 23 has a diameter of 0.1 mm to 0.5 mm. Such diameters enable adequate accommodation of the channels with reduced interference therebetween, allowing them to deliver their intended functions.

As shown in Figs. 3 and 4, in conjunction with Figs. 5a to 5c, there may be two electrodes, for example, which are a proximal electrode 216 and a distal electrode 217. The temperature-control fluid output holes 215 may be provided in both electrodes, preferably, the temperature-control fluid output holes 215 are provided at least in the proximal electrode 216. Through the temperature-control fluid output holes 215 in the proximal electrode 216, the temperature-control fluid is released until it fills the entire space where ablation is to be performed. In the illustrated embodiment, the proximal electrode 216 and the distal electrode 217 are both annular and axially spaced apart from each other, the imaging probe 212 is disposed between the proximal electrode 216 and the distal electrode 217. The gap between the proximal electrode 216 and the distal electrode 217 represents the aforementioned space, in which RF ablation is to be performed on a lesion and monitored by the imaging probe 212 for therapeutic efficacy.

The outer diameter of the proposed interventional catheter should be determined taking into account the diameters of blood vessels that it is intended for use with. For example, the outer diameter of the interventional catheter may range from 1.0 mm to 10.0 mm, which makes it suitable for use with any blood vessel in the human body. Preferably, the outer diameter of the interventional catheter does not exceed 2 mm, which makes it suitable for ablation of smaller plaques. Here, it is to be understood that the outer diameter of the interventional catheter is essentially defined by the catheter body 2, as well as by the coupling portion 4 and tip 3, as detailed below. Typically, the outer diameter of the catheter body 2 is typically equal to that of the coupling portion 4, and the outer diameter of the proximal end of the tip 3 is equal to the outer diameter of the catheter body 2. With this arrangement, the portion of the interventional catheter that is intended to be inserted into a human body is straight and has a smooth outer surface.

In one specific embodiment, as shown in Figs. 4 and 5a to 5c, the catheter body 2 includes one imaging channel 21, one temperature-control fluid channel 22 and a plurality of lead channels. For example, it may include two electrodes, two thermocouples and four lead channels. Two of the lead channels serve as temperature-control lead channels 23, the temperature-control lead channels 23 are configured for arrangement therein of temperature-control leads 5 leading from the thermocouples. The other two lead channels serve as electrode lead channels 24, the electrode lead channels 24 are configured for arrangement of electrode leads 6 therein. The temperature-control fluid channel 22, the temperature-control lead channels 23 and the electrode lead channels 24 are arranged around the imaging channel 21, the imaging channel 21 is disposed at the center of the interventional catheter. The two temperature-control lead channels 23 are arranged in symmetry with respect to the imaging channel 21. Likewise, the two electrode lead channels 24 are also arranged in symmetry with respect to the imaging channel 21. The temperature-control fluid channel 22 and the imaging channel 21 extend in parallel to each other. With this arrangement, light, electricity and heat can be transmitted in a compatible manner without mutual influence or interference.

As shown in Fig. 2, in one embodiment, the interventional catheter further includes a tip 3, the tip 3 is coupled to a distal end of the functional member 210 by the coupling portion 4. The coupling portion 4 is a solid portion serving to couple the functional member 210 to the tip 3. The solid coupling portion 4 closes and seals the distal end of the functional member 210. Preferably, the coupling portion 4 is an elastomer, which reduces the risk of the tip 3 causing damage. The coupling portion 4 may be made of polyurethane or silicone, and the diameter of the coupling portion 4 is equal to that of the catheter body 2. An axial length of the coupling portion 4 should not be excessively long or excessively short. If it is too long, the catheter will distally bend at an increased radius, which can degrade its ability to cross an intended lesion. If the length is too short, the coupling portion 4 may be not able to provide sufficient protection to the tip 3. For these reasons, the axial length of the coupling portion 4 ranges from 1 mm to 10 mm.

In general terms, the tip 3 is a soft, non-invasive structure, which allows the tip to cause less damage to blood vessels or tissue. Preferably, the tip 3 defines a guidewire lumen 31, through which a guidewire can be passed to enable rapid exchange. Providing the guidewire lumen 31 in the tip can facilitate exchange and operation of the interventional catheter while not expanding its overall size. An inner diameter of the guidewire lumen 31 may be determined taking into account a diameter of the guidewire to be passed therethrough. For example, the inner diameter of the guidewire lumen 31 may be 0.1 mm to 2 mm. The size of the tip 3 should not be excessively large. Otherwise, it may be difficult to cross a stenotic lesion. For this reason, the tip 3 is relatively small in size. Additionally, the tip 3 is tapered, the tip 3 should not be axially too long, because it will be sharp and tend to cause damage to blood vessels or tissue when having a long length. On the other hand, if its length is too short, its crossability may be degraded. The axial length of the tip 3 may be 5.0 mm to 50 mm, preferably 20 mm.

In a non-limiting example, use of the proposed interventional catheter may include the steps as follows:
1) Connect the imaging interface 12 to an imaging system, the electrical interface 13 to an energy output device, the fluidic interface 11 to a fluid filling device, and the mechanical interface 14 to the driving member.
2) Deliver the interventional catheter over the guidewire into a diseased blood vessel segment and then activate the imaging system. Once activated, the imaging system captures images of the diseased region, from which locations and compositions of the lesions are identified.
3) After the image-based diagnosis, deliver the functional member 210 to the location of an identified lesion and then activate the energy output device, which then delivers therapeutic energy and/or a therapeutic agent toward the lesion. In this process, the imaging system continuously operates to monitor efficacy of the therapeutic energy or delivery of the therapeutic agent.
4) After the therapeutic treatment of the current lesion is complete, advance the interventional catheter to the next lesion and repeat the above therapeutic process.
5) After therapeutic treatment of the entire diseased region is completed, evaluate the therapeutic efficacy by scanning the whole blood vessel segment.
6) Finally, withdraw the entire interventional catheter over the guidewire. This ends the therapeutic procedure.

The proposed interventional catheter is further described below with reference to specific applications thereof.

In one application, the proposed interventional catheter is an ablation catheter employing optical coherence tomography (OCT) and RF ablation and is used for therapeutic treatment of atherosclerotic plaques.

As shown in Figs. 3, 4 and 5a to 5c, the ablation catheter includes an interface portion 1, a catheter body 2, a tip 3 and a coupling portion 4. The interface portion 1 includes a fluidic interface 11, an imaging interface 12 and an electrical interface 13. The catheter body 2 includes a tubular body with an outer diameter of 1.8 mm and a wall thickness of 0.2 mm, making the catheter suitable for treatment of coronary atherosclerosis. The functional member 210 includes a proximal electrode 216 and a distal electrode 217, which are spaced apart from each other along an axis thereof. Both the proximal electrode 216 and the distal electrode 217 are annular and made of a platinum-iridium alloy. The proximal electrode 216 and the distal electrode 217 are configured to output RF energy and each have a thickness of 0.1 mm and an axial width of 2.0 mm. They are provided with respective associated electrode leads 6 each with a diameter of 200 µm. The proximal electrode 216 and the distal electrode 217 each define 12 temperature-control fluid output holes 215 having a diameter of 90 µm, the temperature-control fluid output holes 215 are evenly arranged circumferentially with respect to the electrode. The tubular body between the proximal electrode 216 and the distal electrode 217 is transparent and serves as an imaging window 214. Moreover, a portion of the tubular body around a proximal end of the proximal electrode 216 may also form part of the imaging window 214. The tubular body defines an imaging channel 21, a temperature-control fluid channel 22, two temperature-control lead channels 23 and two electrode lead channels 24 therein, which are independent of one another. The proximal electrode 216 and the distal electrode 217 are coupled to respective thermocouples provided with respective associated temperature-control leads 5 having a diameter of 200 µm. The imaging channel 21 has a diameter of 0.5 mm. A GRIN lens for receiving and emitting optical signals through the imaging window 214 is provided, as an imaging probe 212, between the proximal electrode 216 and the distal electrode 217. An imaging optical fiber is received in the imaging channel 21 and axially translated and rotated by a driving member therein, concurrently with the GRIN lens emitting and collecting optical signals. The temperature-control fluid channel 22 is arranged beside the imaging channel 21. The temperature-control fluid channel 22 has a diameter of 0.3 mm. A cold saline solution flows from a fluid filling device through the fluidic interface 11 into the temperature-control fluid channel 22. It then further flows through the temperature-control fluid output holes 215 in the electrodes to a lesion in need of RF ablation, thereby cooling down the lesion while not affecting endothelial cells. The cold saline solution can be supplied at an adjustable flow rate, which can satisfy various different cooling requirements. Each electrode is welded to a respective one of the associated electrode leads 6 and to a respective one of the temperature-control leads 5 associated with the respective thermocouples. The electrode leads 6 are made of a platinum-iridium alloy, and the temperature-control leads 5 are made of a copper-nickel alloy. The two temperature-control leads 5 and the two electrode leads 6 are each received in a respective one of the lead channels, which are arranged in symmetry with respect to the imaging channel 21 and the temperature-control fluid channel 22. The imaging interface 12 and the mechanical interface 14 are integrally formed and together coupled to the imaging system. The imaging probe 212 utilizes the imaging optical fiber in combination with a microlens to emit laser light and collects optical signals reflected from a blood vessel. In a surgical procedure, the ablation catheter is advanced over a guidewire running in a guidewire lumen 31 in a tip 3 into the blood vessel. The tip 3 adopts a rapid exchange design, and providing the guidewire lumen 31 in the tip allows for rapid exchange of interventional instruments and enables the catheter to have a reduced overall size. The guidewire lumen 31 has an inner diameter of 0.5 mm, and the tip 3 has an axial length of 20 mm.

Specifically, during use of the ablation catheter, images of the blood vessel are first captured by the OCT system, and plaques are then analyzed for their morphology and composition based on the images. Subsequently, according to the results of the analysis, the two annular electrodes (i.e., the proximal electrode 216 and the distal electrode 217) are aligned with a plaque to be ablated. The RF system (i.e., the energy output device) is activated to apply RF currents through the electrical interface 13 to generate resistive heat to ablate the plaque in the blood vessel. In this process, the imaging probe 212 continuously rotates and collects image signals in real time to monitor progress of the ablation, and the thermocouples on the surfaces of the annular electrodes carry out real-time temperature monitoring. At the same time, the cold saline solution is continuously supplied from the temperature-control fluid output holes 215 to flood the surfaces of the electrodes and the tissue being treated, thus conditioning the temperature and reducing possible damage to endothelial cells. After the ablation of the current plaque is completed, the interventional catheter is moved to the next lesion, and the ablation process is then repeated thereon. After treatment of the last lesion is completed, efficacy evaluation is carried out on the whole blood vessel based on OCT imaging. At last, the catheter is withdrawn, ending the procedure.

It is particularly noted that the ablation catheter is not limited to being an RF ablation catheter and may be alternatively implemented as a cryoablation catheter. In the latter case, the tubular body may define a cryofluid passage therein, and a cryofluid may be jetted from a distal outlet port of the cryofluid passage onto an inner surface of a balloon possible disposed over the functional member. It will be recognized that, so far, there has been no technique for RF ablation of atherosclerotic plaques under the guidance of intravascular imaging and temperature control. The proposed interventional catheter can be made with an outer diameter not greater than 2 mm, making it suitable for thermal ablation of smaller plaques. Combining RF ablation with imaging and temperature monitoring enables more accurate RF ablation with better efficacy.

The medical interventional catheter proposed herein can employ one or more approaches to diagnose and therapeutically treat vascular, cardiac and other diseases. For example, OCT imaging can be used prior to a therapeutic procedure to carry out therapeutic planning. Moreover, OCT imaging and temperature monitoring can be used to provide real-time efficacy feedback during the therapeutic procedure, and to evaluate the overall therapeutic efficacy after the therapeutic procedure is completed, resulting in effective improvements in therapeutic efficacy. In addition, the interventional catheter can be manufactured as a modular component system, in which various functional modules can be incorporated. This overcomes the challenge of deployment and functional delivery in limited spaces and provides for a multi-functional catheter with both diagnostic and therapeutic capabilities, which is expected to provide effectively improved efficacy in clinical applications. The interventional catheter of the present invention can accurately locate a lesion and monitor its therapeutic treatment in real time, simplifying the therapeutic procedure and facilitating the surgeon's operation. Further, the interventional catheter can be repeatedly used in a therapeutic procedure until all the identified lesions are therapeutically treated, resulting in material savings, helping reduce the incidence of complications that conventional interventional surgery often suffers from, improving clinical outcomes, lowering the rate of re-hospitalization, and relieving household burdens and social and economic losses incurred by diseases. Therefore, it is beneficial in terms of economy and ecology.

Although the present invention has been disclosed hereinabove, it is not limited to the above disclosure. Those skilled in the art can make various changes and modifications to the invention without departing from the spirit and scope thereof. Accordingly, it is intended that any and all such changes and modifications also fall within the scope of the present invention as defined by the appended claims and equivalents thereof.

## Claims

1. A medical interventional catheter, comprising a catheter body with both diagnostic and therapeutic capabilities, the catheter body comprising a functional member at a distal end thereof, the functional member configured for image-based monitoring of a tubular lumen of interest and for release of a therapeutic source to a target location in the tubular lumen of interest, the therapeutic source comprising therapeutic energy and/or a therapeutic substance.

2. The medical interventional catheter according to claim 1, further comprising a tip, wherein a distal end of the functional member is connected to a proximal end of the tip by an elastic coupling portion, the distal end of the functional member is sealed by the elastic coupling portion, and the tip defines a guidewire lumen.

3. The medical interventional catheter according to claim 1 or 2, wherein the functional member accomplishes the image-based monitoring of the tubular lumen of interest using one or more imaging approaches.

4. The medical interventional catheter according to claim 3, wherein the functional member accomplishes the image-based monitoring of the tubular lumen of interest using optical coherence tomography imaging.

5. The medical interventional catheter according to claim 4, wherein the functional member comprises an imaging probe and a transparent imaging window, the imaging probe disposed at the transparent imaging window.

6. The medical interventional catheter according to claim 1 or 2, wherein the functional member is able to release one or more types of therapeutic energy.

7. The medical interventional catheter according to claim 6, wherein the functional member is able to release at least one of radio frequency (RF) radiation, ultrasound waves, laser light and a cryofluid as the therapeutic energy.

8. The medical interventional catheter according to claim 1 or 2, wherein the functional member accomplishes the release of the therapeutic substance to the target location in the tubular lumen of interest using one or more approaches.

9. The medical interventional catheter according to claim 8, wherein the therapeutic substance is a drug, wherein the functional member accomplishes the release of the therapeutic substance to the target location in the tubular lumen of interest using at least one of a drug-containing coating, a drug-delivery reservoir and drug-delivery microneedles.

10. The medical interventional catheter according to claim 9, wherein the functional member employs a drug-delivery reservoir and/or drug-delivery microneedles, wherein the catheter body further comprises a tubular body having an outer surface, on which the drug-delivery reservoir and/or the drug-delivery microneedles is/are provided, the catheter body defining therein a drug-delivery channel extending along an axis of the tubular body of the functional member to a proximal end of the tubular body and a distal end of the drug-delivery channel connected to the drug-delivery reservoir and/or the drug-delivery microneedles.

11. The medical interventional catheter according to claim 1 or 2, wherein the functional member comprises an imaging probe, an electrode and a temperature-measuring element, the imaging probe configured to accomplish the image-based monitoring, the electrode configured to release RF radiation, the temperature-measuring element disposed on the electrode and configured to monitor a surface temperature of the electrode.

12. The medical interventional catheter according to claim 11, wherein the catheter body further comprises a tubular body, wherein the imaging probe is provided in the tubular body, and the electrode is disposed on an outer surface of the tubular body,
the tubular body defines an imaging channel, an electrode lead channel and a temperature-control lead channel therein, which are independent of one another and each extend along an axis of the tubular body of the functional member to a proximal end of the tubular body, the imaging channel arranged at a center of the tubular body, the electrode lead channel and the temperature-control lead channel both arranged around the imaging channel,
the imaging channel provided therein with an imaging transmission structure connected to the imaging probe, the electrode lead channel provided therein with an electrode lead connected to the electrode, the temperature-control lead channel provided therein with a temperature-control lead connected to the temperature-measuring element.

13. The medical interventional catheter according to claim 12, wherein the tubular body further defines a temperature-control fluid channel therein, which is independent of the imaging channel, the electrode lead channel and the temperature-control lead channel and arranged beside the imaging channel, wherein the electrode defines temperature-control fluid output holes, and the temperature-control fluid channel extends from the proximal end of the tubular body along the axis thereof and is connected to the temperature-control fluid output holes, and the temperature-control fluid channel configured for transfer of a temperature-control fluid and for release of the temperature-control fluid to the target location through the temperature-control fluid output holes.

14. The medical interventional catheter according to claim 13, wherein the tubular body is a braided single-lumen tube, the braided single-lumen tube housing therein a temperature-control fluid tube, an imaging tube, an electrode lead tube and a temperature-control lead tube, the temperature-control fluid tube defining a lumen providing the temperature-control fluid channel, the imaging tube defining a lumen providing the imaging channel, the electrode lead tube defining a lumen providing the electrode lead channel, the temperature-control lead tube defining a lumen providing the temperature-control lead channel, each of the temperature-control fluid tube, the imaging tube, the electrode lead tube and the temperature-control lead tube having a wall thickness less than 0.2 mm.

15. The medical interventional catheter according to claim 14, wherein the temperature-control fluid tube, the imaging tube, the electrode lead tube and the temperature-control lead tube are securely bonded together.

16. The medical interventional catheter according to claim 13, wherein the tubular body has an outer diameter of 1.0 mm to 3.0 mm, the imaging channel has a diameter not exceeding 1.0 mm, the temperature-control fluid channel has a diameter not exceeding 0.5 mm, the electrode lead channel has a diameter of 0.1 mm to 0.5 mm, and the temperature-control lead channel has a diameter of 0.1 mm to 0.5 mm.

17. The medical interventional catheter according to claim 13, wherein the temperature-control fluid output holes are micro-holes, the micro-holes having a diameter of 50 µm to 200 µm.

18. The medical interventional catheter according to claim 11, wherein a plurality of electrodes are provided, the plurality of electrodes are spaced axially and/or circumferentially with respect to the catheter body, at least two of the plurality of electrodes are annular and spaced axially with respect to the catheter body, wherein the imaging probe is provided between adjacent two of the annular electrodes.

19. The medical interventional catheter according to claim 13, further comprising an interface portion, wherein a proximal end of the catheter body is connected to the interface portion, and the interface portion comprises:
an imaging interface connected to a proximal end of the imaging transmission structure;
a fluidic interface connected to a proximal end of the temperature-control fluid channel; and
an electrical interface connected to proximal ends of the electrode lead and the temperature-control lead.

20. The medical interventional catheter according to claim 1 or 2, wherein the functional member comprises an imaging probe configured to accomplish the image-based monitoring, wherein the catheter body further comprises a tubular body, the imaging probe is disposed in the tubular body, the tubular body defining therein an imaging channel, the imaging channel extending along an axis of the tubular body of the functional member to a proximal end of the tubular body, the imaging channel provided therein with an imaging transmission structure connected to the imaging probe, the imaging transmission structure configured to be driven by a driving member to rotate the imaging probe circumferentially and/or translate the imaging probe axially with respect to the catheter body.

21. The medical interventional catheter according to claim 20, wherein the imaging transmission structure comprises an imaging optical fiber, a protective tube and a torsion spring, the protective tube disposed over the imaging optical fiber, the torsion spring disposed between the protective tube and the imaging optical fiber, one end of the imaging optical fiber being connected to the imaging probe and the other end thereof being connected to the driving member.
